# EUROPEAN PATENT APPLICATION

(11) **EP 2 336 926 A2**
(43) Date of publication of application: **22.06.2011**
(21) Application number: 11002722.4
(22) Date of filing: 30.05.2007
(51) Int. Cl.: G06F 19/00

(54) **Patient monitoring system with contextual help**

(30) Priority: 31.05.2006 US 445495
(62) Divisional of application: 07795641.5
(71) Applicant: Nellcor Puritan Bennett LLC, Pleasanton, CA 94588 (US)
(72) Inventor: Wang Hui, San Ramon CA 94582 (US)
(74) Representative: Kinsler, Maureen Catherine

(57) **Abstract**

A monitoring system, comprising: a patient monitor comprising a display adapted to provide a plurality of output displays of computerized processes, wherein the plurality of output displays are adapted to be associated with a respective plurality of contexts; and a user input device in communication with the monitor, wherein the user input device is configured to effect activation of (a) a plurality of help screens on the display, wherein the plurality of help screens are adapted to be associated with the respective plurality of contexts, and wherein at least one of the help screens comprises selectable text or (b) a plurality of audio help messages, wherein the plurality of help messages are adapted to be associated with the respective plurality of contexts.

## Description

### 1. Technical Field

The present invention relates generally to help screen systems for patient physiological data monitoring instruments. In particular, the present invention relates to a context-based help screen system including a main menu of help topics and subscreens of help information.

### 2. Background Art

This section is intended to introduce the reader to various aspects of art that may be related to various aspects of the present invention, which are described and/or claimed below. This discussion is believed to be helpful in providing the reader with background information to facilitate a better understanding of the various aspects of the present invention. Accordingly, it should be understood that these statements are to be read in this light, and not as admissions of prior art.

In the field of medicine, doctors often desire to monitor certain physiological characteristics of their patients. Accordingly, a wide variety of devices have been developed for monitoring many such characteristics of a patient. Such devices provide doctors and other healthcare personnel with the information they need to provide the best possible healthcare for their patients. As a result, such monitoring devices have become an indispensable part of modern medicine.

One technique for monitoring certain physiological characteristics of a patient is commonly referred to as pulse oximetry, and the devices built based upon pulse oximetry techniques are commonly referred to as pulse oximeters. Pulse oximetry may be used to measure various blood flow characteristics, such as the blood-oxygen saturation of hemoglobin in arterial blood, the volume of individual blood pulsations supplying the tissue, and/or the rate of blood pulsations corresponding to each heartbeat of a patient.

Pulse oximetry typically utilizes a patient monitoring device that, among other functions, displays information related to patient vital signs and provides an audible and/or visual alarm when changes in the vital signs so warrant. This improves patient care by facilitating continuous supervision of a patient without continuous attendance by a human observer (e.g., a nurse or physician).

As pulse oximetry has become more sophisticated, the number and variety of functions that a pulse oximetry monitor may perform has increased. Thus, operating a pulse oximetry monitor has become more complex for the user. Although pulse oximetry monitors typically have detailed user manuals, these manuals may not always be stored together with the monitoring instruments. It would be desirable to provide a user help system with a pulse oximetry monitor that is integral to the device. This would allow a healthcare provider to immediately access the system to answer any questions about the operation of the device without losing time searching for the user manual.

### DISCLOSURE OF THE INVENTION

Certain aspects commensurate in scope with the originally claimed invention are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

There is provided a monitoring system that includes: a patient monitor adapted to display physiological information related to a patient; and a user input device in communication with the monitor, wherein the user input device is configured to cause the monitor to display a help screen including selectable text.

There is also provided a monitoring system that includes: a patient monitor comprising a display adapted to provide a plurality of output displays of computerized processes, wherein the plurality of output displays are adapted to be associated with a respective plurality of contexts; and a user input device in communication with the monitor, wherein the user input device is configured to effect activation of a plurality of help screens on the display, wherein the plurality of help screens are adapted to be associated with the respective plurality of contexts and wherein at least one help screen includes selectable text.

There is also provided a monitor that is configured to display physiological data from a patient that includes a first help screen comprising selectable text related to a first displayed physiological information, wherein the selectable text is adapted to effect a display of a second help screen related to a second displayed physiological information.

There is also provided a computer-readable medium comprising: code adapted to generate information related to the physiological state of a patient; code adapted to display a first help screen comprising selectable text, wherein the help screen is related to the information; code adapted to receive one or more input signals from a user-input device adapted to select the selectable text; and code adapted to display a second help screen upon receipt of the input signal from the user-input device.

There is also provided a monitoring system that includes a patient monitor adapted to display physiological information related to a patient; and a user input device in communication with the monitor, wherein the user input device is configured to cause the monitor to display a help screen associated with an audible help message.

There is also provided a monitoring system that includes a patient monitor comprising a display adapted to provide a plurality of output displays of computerized processes, wherein the plurality of output displays are adapted to be associated with a respective plurality of contexts; and a user input device in communication with the monitor, wherein the user input device is configured to effect activation of a plurality of audio help messages, wherein the plurality of help messages are adapted to be associated with the respective plurality of contexts.

### BRIEF DESCRIPTION OF DRAWINGS

Advantages of the invention may become apparent upon reading the following detailed description and upon reference to the drawings in which:
FIG. 1 is a perspective view of a patient monitor in accordance with an exemplary embodiment of the present invention;
FIG. 2A is a view of an exemplary help screen menu in accordance with an exemplary embodiment of the present invention;
FIG. 2B is a view of an exemplary help screen menu in accordance with an exemplary embodiment of the present invention;
FIG. 3 is a is a view of an exemplary help subscreen in accordance with an exemplary embodiment of the present invention;
FIG. 4 is a is a view of an exemplary help screen in accordance with an exemplary embodiment of the present invention;
FIG. 5 is a view of an exemplary display screen showing that a help screen may be accessed from a soft menu in accordance with an exemplary embodiment of the present invention;
FIG. 6 is a flow chart of a method for providing a context-based help screen in accordance with an exemplary embodiment of the present invention;
FIG. 7 is a block diagram of an audio help module for providing audio versions of help screens in accordance with an exemplary embodiment of the present invention; and
FIG. 8 is a view of a multiparameter monitor and exemplary patient monitor in accordance with the present techniques.

### MODES FOR CARRYING OUT THE INVENTION

One or more specific embodiments of the present invention will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

The present techniques relate to a patient monitor with a help screen system. A patient monitor, such as a pulse oximeter, is provided that displays physiological information and includes a user-input device that allows a user to access a help screen system.

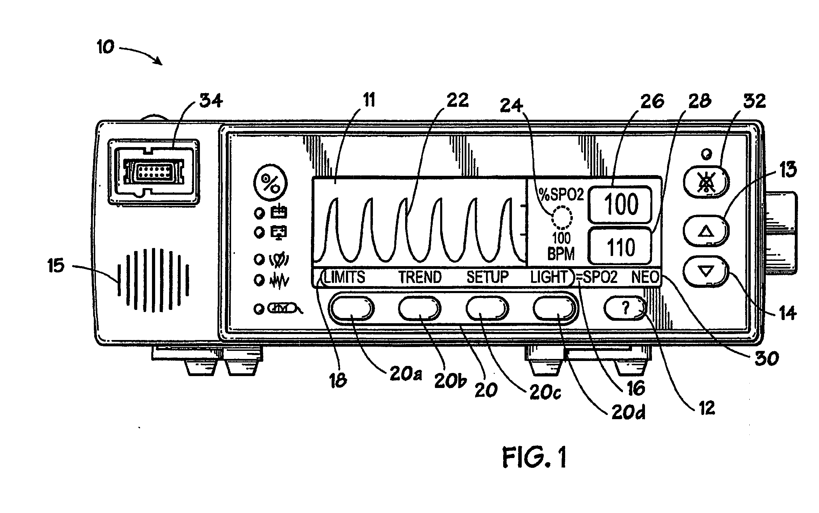

Fig. 1 is a perspective view of a patient monitor 10 in accordance with an exemplary embodiment of the present invention. The monitor 10 includes a display window 11, which may be a cathode ray tube or liquid crystal display, for example. The display window 11 is coupled with the monitor 10 and may display physiological data and other information. For example, the monitor 10 may be a pulse oximeter, such as those available from Nellcor Puritan Bennett LLC. The monitor 10 includes a user-input device for activating a help screen display that may include any type of user-input mechanism, such as a fixed function key, a soft key, a remote activation device, a touch screen, or a voice recognition device. An exemplary user-input device is depicted here as a help key 12 that effects the display of a help screen in the display window 11, discussed in more detail below. The help key 12 may include a question mark icon or a text indicator such as HELP. In certain embodiments, activation of the help key 12 may involve pressing or pressing the key 12 and releasing the key 12. Activation of the help key 12 may switch the display window 11 view from a patient information display to a help screen display. Such a switch may involve complete replacement of the previous display, or may involve only partial replacement of the display such that sections of the previous display remain visible. In certain embodiments (not shown) the monitor 10 may include an additional display window that is a dedicated help display window. In such an embodiment, the help screen may be displayed without interfering with the display from the first output display window.

In general, the monitor 10 includes functions such as processing physiological data and/or other data received from a patient sensor (discussed below) via a cable connection port 34 that is configured to communicatively couple with the sensor. The monitor 10 may be processor-based and software-controlled. The software may be stored in memory, such as RAM, ROM, flash, or on ASIC. Additionally, the monitor 10 may be re-programmed. The processed data may be displayed in the display window 11. For example, a display may include a plethysmographic ("pleth") waveform display 22, an oxygen saturation display 26, and/or a pulse rate display 28 that are displayed in a main display screen or a pleth display screen. The oxygen saturation displays may be a functional arterial hemoglobin oxygen saturation measurement in units of percentage SpO₂. The pulse rate display 28 may indicate a patient's pulse rate in beats per minute. In other embodiments, the display window 11 may show an initial display immediately after the monitor 10 is turned on that includes the general monitor information, such as the serial number of the instrument and the software version. In other embodiments, the display window 11 may show topic-specific screens, such as a setup display, a "blip" display that includes pulse amplitude blips, a real-time trend display, and an alarm limit and monitoring mode display.

In addition to displaying physiological information, the monitor 10 may also display information related to alarms and monitor settings. The monitor also may include a speaker 15 for audible alarm signals. For example, in some embodiments, the monitor 10 employs SatSeconds™ by Nellcor™ to detect alarms and manage nuisance alarms. SatSeconds™ may include activation of an alarm based on limits that may include the integral of time and depth of a desaturation event and may also include an indicator 24 that may serve to inform the operator that an SpO₂ reading has been detected outside of the limit settings. The display may also include an alarm status indicator (not shown), such as a bell that flashes when an alarm condition is present. When the alarm is silenced using the alarm silence button 32, an alarm silence indicator, such as a slash and a timer, may be shown to indicate that the alarm is temporarily silenced. When the alarm is silenced through an "all mute" menu selection, which is permanent until power is cycled or deselected using menu, an alarm status indicator with a slash may shown to indicate that alarm has been silenced. Further, the display may include mode setting information such as neonatal mode alarm limits or adult mode alarm limits indicators 30 and special settings such as a fast response mode setting indicator 16.

In addition to a help key 12, or other help user-input device, the monitor 10 may include a number of keys that are related to the operating functions. The keys may include fixed function keys, such as the arrow up key 13 or the arrow down key 14 that may be used to scroll through items in the display window 11. Fixed function keys may be configured to have dual functions. For example, in certain embodiments (not shown), the help key 12 may be configured to bring up a help screen when depressed for less than a predetermined amount of time, and may be used to adjust the contrast in combination with the arrow up key 13 and arrow down key 14 when pressed for longer than the predetermined amount of time. In such an embodiment where the help key 12 includes a programmed contrast adjust function, the help key 12 may include two different icons, such as a question mark icon and a light bulb icon. The monitor 10 may also include programmable function keys ("soft keys") 20, and associated soft key icons in the soft key menu 18. The four soft keys 20a, 20b, 20c, and 20d are pressed to select a corresponding one of the soft key icons. The soft key icon menu 18 indicates which software menu items can be selected through the soft keys 20. Pressing a soft key 20 associated with, such as below, above, or next to an icon, selects the option.

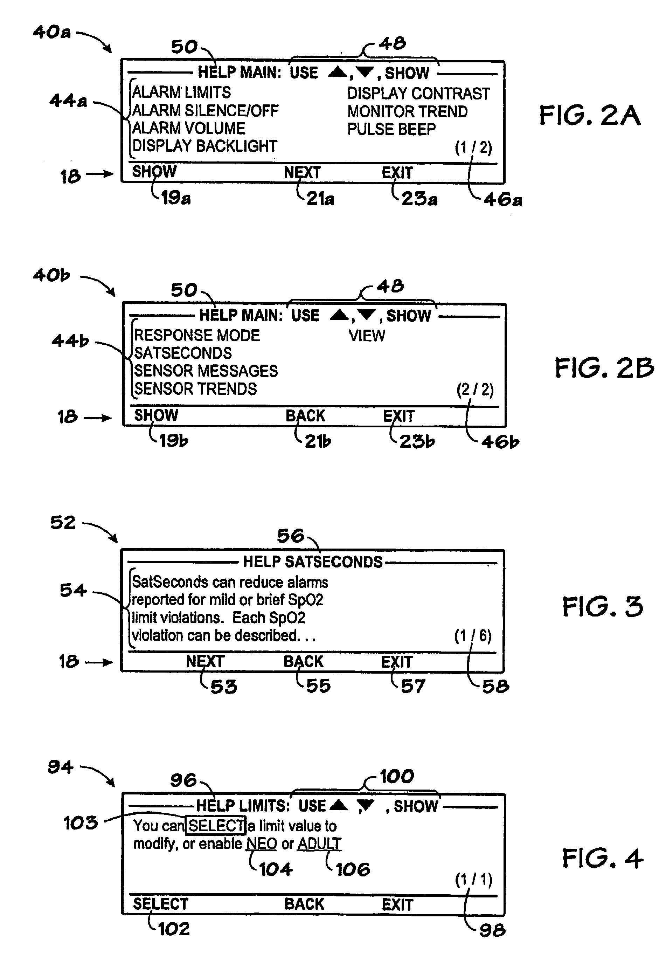

Pressing the help key 12 while in the pleth or main display may effect the display of a help screen main menu 40, described in more detail in Fig. 2A and Fig. 2B. The help screen main menu 40 may include one or more pages or screens of help topics 44 that may be accessed in subscreens or submenus from the help screen main menu 40. The help screen main menu may include help topics such as alarms limits, SatSeconds™, alarm volume, alarm silence/off, pulse beep/volume, main view, monitor trends, sensor event trends, display contrast, sensor messages, response mode, or display back light brightness. It is envisioned that the help screen system as provided herein may be hierarchical, with a main help screen and multiple associated subscreens. For example, each of the above help screen topics 44 may be selected from the help screen main menu 40. Once selected, these topics are accessed via subscreens that may include general help information or further help submenus of specific topics. Further, the help screen may include text information. For example, a help screen may include text explanation of an icon on a display or on a user key or button. A help screen may also include instructions on setting up the monitor 10 or adjusting various settings.

The help screen main menu 40 may include an indicator 50 that informs the operator of the name of screen display in the display window. Further, the help screen main menu 40 may also include a function key indictor 48 that informs the operator which fixed function keys or soft keys may be used to navigate through the help screen main menu 40. As depicted in Fig. 2A and Fig. 2B, the fixed keys may include the arrow up key 13 or the arrow down key 14. Depressing the arrow up key 13 or arrow down key 14 may allow an operator to scroll through the help topics 44. Individual help topics may be highlighted as the operator scrolls through them. The soft keys in use on the help screen main menu 40 display may be located in the menu bar 18, and may include the SHOW soft key icon 19b. Thus, if an operator wishes to access the alarm volume help subscreen, he may use the arrow down key 14 and access the topic that is highlighted by depressing the soft key associated with the SHOW soft key icon 19a. As depicted, there are two pages of pages of help topics 44, shown as 44a in Fig. 2A and 44b in Fig. 2B, as an operator may note by observing the page indicators 46a and 46b. An operator may access the second page of the help screen main menu 40b from the first page of the help screen main menu 40a by depressing the soft key associated with the NEXT soft key icon 21a. From the second page of the help screen main menu 40b, the operator may navigate back to the first page by depressing the soft key associated with the BACK soft key icon 21b. Alternatively, an operator may access the second page of the help screen mean menu 40b from the first page of the help screen main menu 40a by depressing the fixed arrow up key 13 or the arrow down key 14 after the first or last topic on the first page, respectively, has been selected using these arrow keys. Similarly, an operator may navigate back to the first page of the help screen mean menu 40a from the second page of the help screen main menu 40b by depressing the fixed arrow up key 13 or the arrow down key 14 after the first or last topic on the second page, respectively, has been selected using these arrow keys. The operator may exit the help screen main menu 40 at any point by depressing the soft key associated with the EXIT soft key icon 23a or 23b.

Fig. 3 illustrates an exemplary topic-specific SatSeconds™ help screen 52. As discussed above, the SatSeconds™ help screen 52 may be accessed from the help main menu 40. For example, after pressing the help key 12 to display the help main menu, an operator may scroll through the available help topics 44 or may also press the soft key associated with the NEXT soft key icon 21a to access page (2 / 2). The SatSeconds™ help screen 52 may include a name display 56 as well as a page number display 58. As depicted, the SatSeconds™ help screen 52 may include several pages of help information 54. The operator may access subsequent help information 54 pages by using the soft key associated with the NEXT soft key icon 53 or the BACK soft key icon 55. Upon using the soft key associated with the EXIT soft key icon 57, the display will return to the SAT-S screen. If help screen 52 is the first or only page available, the BACK soft key icon 55 may also return the display to the SAT-S screen. In other embodiments, actuating the help key 12 from the help screen 52 may also return the display to a previous screen, such as a SAT-S screen. Further, the help screen may timeout at a desired time to return to the previous monitor display, such as a physiological data display. In certain embodiments, the runtime software switches to a main menu or previous display when an alarm registers. In other embodiments, an alarm message may be overwritten over a help screen 52. In such an embodiment, the operator may actuate the help key 12 to exit the help screen 52 and return back to the previous display.

In an alternate embodiment shown in Fig. 4, a topic-specific help screen may be accessed in a context-dependent manner directly from a monitor 10 display. In this embodiment, an operator may depress the soft key 20a (see Fig. 1) associated with the LIMITS soft key icon on the monitor 10 soft key menu bar 18 to display the limits menu (not shown). From the limits menu (not shown), the operator may then depress the help key 12 to display a limits help screen 94. The limits help screen 94 may include a name indicator 96 identifying the screen as well as a page number display 98, indicating that the limits help 94 includes a single screen. However, it should be understood that the help screens described herein include any suitable number of screens and the limits help screen 94 depicted here is merely exemplary.

As depicted, the limits help screen 94 may be formatted with rich text to include any suitable number of hyperlinks to limits help subscreens. The hyperlinks may be embedded in selectable text. For example, an operator may access the various subscreens by using the function keys shown in the function key indicator 100. An operator may use the arrow up key 13 or arrow down key 14 to scroll through the available hyperlinks, such as the NEO hyperlink 104 and ADULT hyperlink 106. The operator may also access a limits help subscreen by depressing the soft key associated with the SHOW soft key icon 102. The presence of the NEO hyperlink 104 and ADULT hyperlink 106 within the text body may be indicated by default underlining. Alternatively, an operator may depress a SELECT softkey 102 to scroll through the selectable regions using the up arrow 13 or the down arrow 14. Upon depressing the SELECT softkey 102 the text "Select" in the help screen 94 may be highlighted, and the NEO hyperlink 104 and ADULT hyperlink 106 may then be displayed with underlining. In certain embodiment, the selectable text hyperlinks are embedded within the text itself. This may be advantageous when formatting help screens in different languages. In such an embodiment, the screens corresponding to various languages may be parsed into lines according to a predetermined rule. As such, the selectable portions of the text may be associated with the appropriate word in each language. Rich text formatting may also allow for bitmaps and other suitable graphics.

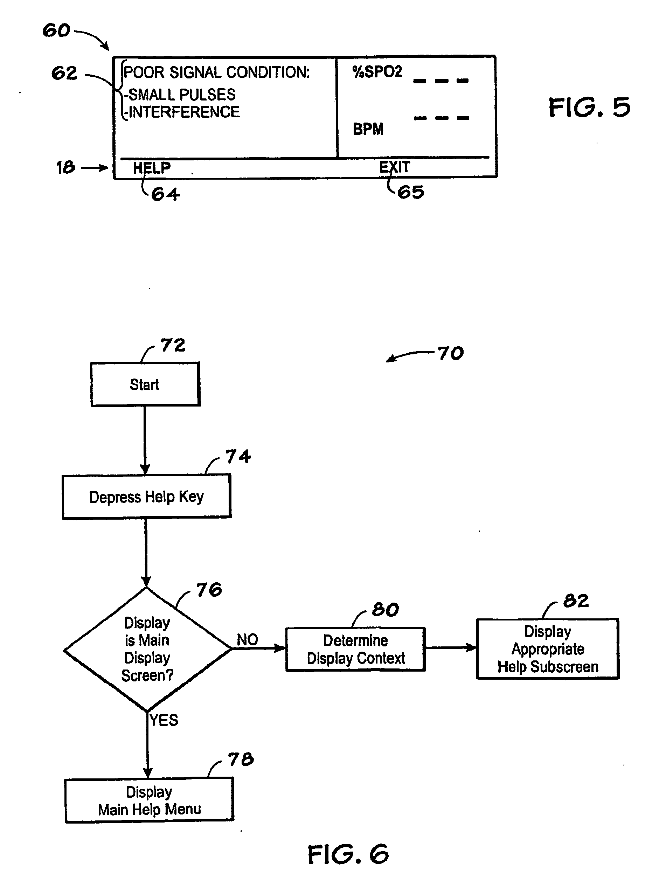

In other embodiments, certain monitor display screens may include access to a help screen from a soft key that is distinct from the designated user-input device for activating the help screen system, for example, the designated help key 12. This may be useful for informing the operator that topic-specific help is available from a particular display. For example, Fig. 5 illustrates an exemplary sensor signal condition display 60. The sensor signal condition display 60 may activate during conditions when the monitor 10 senses that the quality of the sensor signal has fallen below a certain predetermined threshold. As the sensor signal condition display 60 may automatically activate under predetermined conditions without any input from the operator, it may be advantageous to provide help information to the operator to provide further information about the signal condition messages 62. Although a user may also access help screens relating to sensor signals by pressing the help key 12, a soft key menu bar 18 including a help soft key icon 64 may remind the operator that further information for a particular display screen is provided.

Fig. 6 is a block diagram of a method 70 for providing a monitor 10 with context-based help in accordance with an exemplary embodiment of the present invention. Context-based help allows an operator to quickly access help screens relating to topics that are specific to a particular monitor display. Thus, when a user is on a display screen relating to a particular topic, such as alarm management, pressing the help key 12 will bring up the alarm management help screen. The method begins at block 72 and proceeds to block 74, in which a user activates a help key 12. Upon activation of the help key 12, a processor may determine whether a monitor display screen is associated with a main display screen context in block 76. An association of the monitor display with a main display context causes the processor to effect the display of a help screen main menu in block 78. If the display is associated with a particular help context, as determined in block 80, a help screen submenu is displayed in block 82 that is associated with the particular help context. For example, if the monitor is in a trend display, a processor may determine that the trend display is associated with a trend display help context. Upon pressing the help key 12, the monitor display will switch to a trend help subscreen that contains trend help information. Thus, the operator may quickly access individual help topics from certain monitor displays without the necessity of navigating through the help screen main menu. In situations where the monitor display is not associated with a particular help subscreen, the processor may effect the display of the default help screen, the help main menu screen.

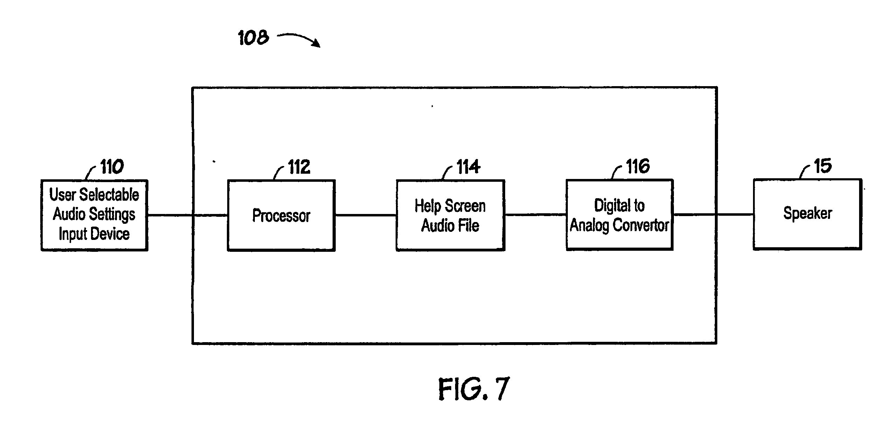

In certain embodiments, the monitor 10 may be configured to provide audible help messages. Audible help messages may be advantageous in for training purposes. In Fig. 7, an audio help module 108 is depicted that is responsive to an audible settings-input device 110. It is envisioned that the monitor settings menus may provide a soft key or other user-input device in order to activate audio help. The audible settings-input device 110 is in communication with a processor 112. The processor 112 is able to retrieve from a memory an appropriate stored audio help file 114 that corresponds with the help screen on the display 11. Audio help files 114 may include mp3 files, WAV files, or any other appropriate digital storage format. A digital to analog converter 116 generates an analog signal from the audio help file 114 and sends the signal to the speaker 15. It is envisioned that the audio help files 114 may contain an audible version of the help screen on the display 11. Further, it is envisioned that the audio help files 114 may contain additional help information that may supplement the text or graphic help information. In certain embodiments, the audio help message may be activated separately from the display of a help screen 52. In such an embodiment, the audio help message may be independent of a particular help screen context. In certain embodiments, the help information may be text, audio, or a combination of the three formats.

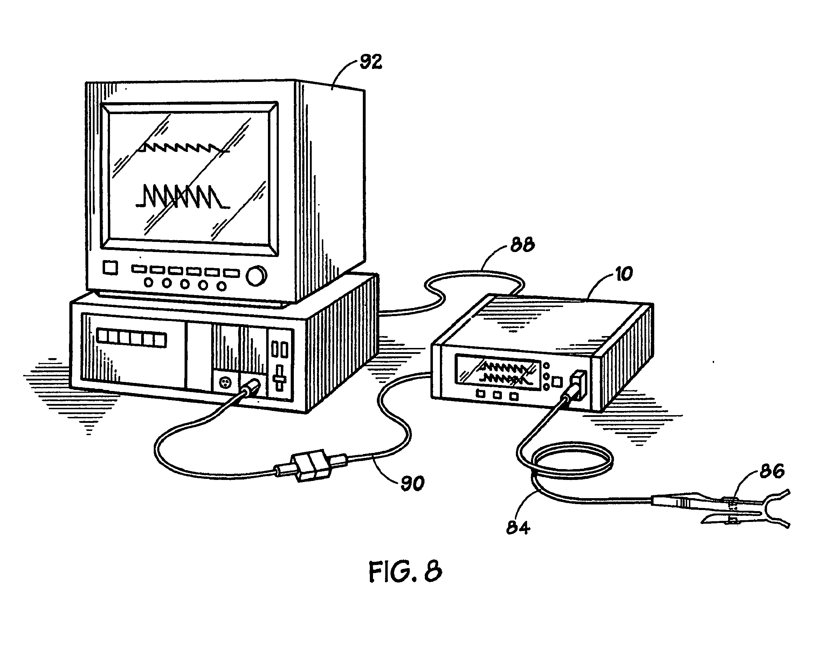

The exemplary pulse oximetry monitor 10 described herein may be used with a sensor 86, as illustrated in Fig. 8. It should be appreciated that the cable 84 of the sensor 86 may be coupled to the monitor 10 or it may be coupled to a transmission device (not shown) to facilitate wireless transmission between the sensor 86 and the monitor 10. The sensor 86 may be any suitable sensor 86, such as those available from Nellcor Puritan Bennett Inc. Furthermore, to upgrade conventional pulse oximetry provided by the monitor 10 to provide additional functions, the monitor 10 may be coupled to a multi-parameter patient monitor 92 via a cable 90 connected to a sensor input port or via a cable 88 connected to a digital communication port. It should be understood that the help screens described herein may be upgraded through, for example, software upgrades or plug-ins that may enhance or alter the help screen displays.

While the invention may be susceptible to various modifications and alternative forms, specific embodiments have been shown by way of example in the drawings and will be described in detail herein. However, it should be understood that the invention is not intended to be limited to the particular forms disclosed. Rather, the invention is to cover all modifications, equivalents and alternatives falling within the spirit and scope of the invention as defined by the following appended clauses.

The following clauses are included in and form part of the description of the invention.
Clause 1: A monitoring system, comprising: a patient monitor adapted to display physiological information related to a patient; and a user input device in communication with the monitor, wherein the user input device is configured to cause the monitor to display a help screen comprising selectable text.
Clause 2: The system of clause 1, wherein the user input device comprises a key or button.
Clause 3: The system of clause 2, wherein the user input device comprises a help icon.
Clause 4: The system of clause 1, wherein monitor is configured to display a plethysmographic waveform, a heart rate, an SpO₂ value, an alarm indicator, or any combination thereof.
Clause 5: The system of clause 1, wherein the selectable text is formatted in rich text.
Clause 6: The system of clause 1, wherein the selectable text comprises a hyperlink.
Clause 7: The system of clause 1, comprising a second user input device adapted to cause the monitor to display a help subscreen.
Clause 8: The system of clause 1, wherein the help screen comprises at least two pages of help screens.
Clause 9: The system of clause 8, comprising a second user input device adapted to effect a switch between the at least two pages of help screens.
Clause 10: The system of clause 1, comprising a second user input device adapted to close the help screen.
Clause 11: The system of clause 1, wherein the first-user input device is adapted to effect a switch from the display of the physiological information to the help screen.
Clause 12: The system of clause 1, comprising a first display window adapted to display the physiological information and a second display window adapted to display the help screen.
Clause 13: The system of clause 1, wherein the patient monitor comprises a pulse oximeter.
Clause 14: A monitoring system, comprising: a patient monitor comprising a display adapted to provide a plurality of output displays of computerized processes, wherein the plurality of output displays are adapted to be associated with a respective plurality of contexts; and a user input device in communication with the monitor, wherein the user input device is configured to effect activation of a plurality of help screens on the display, wherein the plurality of help screens are adapted to be associated with the respective plurality of contexts, and wherein at least one of the help screens comprises selectable text.
Clause 15: The system of clause 14, wherein the user input device comprises a key or button.
Clause 16: The system of clause 14, wherein the user input device comprises a help icon.
Clause 17: The system of clause 14, wherein at least one of the output displays comprises a main display associated with a main display context.
Clause 18: The system of clause 14, wherein the plurality of output displays comprises a plethysmographic waveform display associated with a main display context.
Clause 19: The system of clause 18, wherein the help screen associated with the plethysmographic waveform display context comprises a help screen main menu. Clause 20: The system of clause 19, wherein the help screen main menu comprises at least one help topic.
Clause 21: The system of clause 14, wherein the plurality of output displays comprise a sensor signal condition display associated with a signal condition display context.
Clause 22: The system of clause 21, wherein the help screen associated with the sensor signal condition display context comprises a sensor help screen.
Clause 23: The system of clause 14, wherein the patient monitor comprises a pulse oximeter.
Clause 24: A monitor configured to display physiological data from a patient, the monitor comprising: a first help screen comprising selectable text related to a first displayed physiological information, wherein the selectable text is adapted to effect a display of a second help screen related to a second displayed physiological information.
Clause 25: The monitor of clause 24, wherein the first help screen and the second help screen are not displayed at the same time.
Clause 26: The monitor of clause 24, wherein the monitor comprises a pulse oximeter.
Clause 27: A computer-readable medium comprising: code adapted to generate information related to the physiological state of a patient; code adapted to display a first help screen comprising selectable text, wherein the help screen is related to the information; code adapted to receive one or more input signals from a user-input device adapted to select the selectable text; and code adapted to display a second help screen upon receipt of the input signal from the user-input device.
Clause 28: The computer readable medium of clause 27, wherein the series of help screens is hierarchical.
Clause 29: A monitoring system, comprising: a patient monitor adapted to display physiological information related to a patient; and a user input device in communication with the monitor, wherein the user input device is configured to cause the monitor to display a help screen associated with an audible help message.
Clause 30: The system of clause 29, wherein the user input device comprises a key or button.
Clause 31: The system of clause 29, wherein the user input device comprises a help icon.
Clause 32: A monitoring system, comprising: a patient monitor comprising a display adapted to provide a plurality of output displays of computerized processes, wherein the plurality of output displays are adapted to be associated with a respective plurality of contexts; and a user input device in communication with the monitor, wherein the user input device is configured to effect activation of a plurality of audio help messages, wherein the plurality of help messages are adapted to be associated with the respective plurality of contexts.
Clause 33: The system of clause 32, wherein the user input device comprises a key or button.
Clause 34: The system of clause 32, wherein the user input device comprises a help icon.
Clause 35: The system of clause 32, wherein at least one of the output displays comprises a main display associated with a main display context.

## Claims

1. A monitoring system, comprising: a patient monitor comprising a display adapted to provide a plurality of output displays of computerized processes, wherein the plurality of output displays are adapted to be associated with a respective plurality of contexts; and a user input device in communication with the monitor, wherein the user input device is configured to effect activation of (a) a plurality of help screens on the display, wherein the plurality of help screens are adapted to be associated with the respective plurality of contexts, and wherein at least one of the help screens comprises selectable text or (b) a plurality of audio help messages, wherein the plurality of help messages are adapted to be associated with the respective plurality of contexts.

2. The system of claim 1, wherein the user input device comprises a key or button or a help icon.

3. The system of claim 1, wherein at least one of the output displays comprises a main display associated with a main display context.

4. The system of claim 1, wherein the plurality of output displays comprises a plethysmographic waveform display associated with a main display context.

5. The system of claim 4, wherein the help screen associated with the plethysmographic waveform display context comprises a help screen main menu.

6. The system of claim 5, wherein the help screen main menu comprises at least one help topic.

7. The system of claim 1, wherein the plurality of output displays comprises a sensor signal condition display associated with a signal condition display context.

8. The system of claim 7, wherein the help screen associated with the sensor signal condition display context comprises a sensor help screen.

9. The system of any of the preceding claims, wherein the patient monitor is configured to display a plethysmographic waveform, a heart rate, a SpO₂ value, an alarm indicator, or any combination thereof.

10. The system of any of the preceding claims, wherein the patient monitor comprises a pulse oximeter.
